(19) Europäisches Patentamt
European Patent Office
Office européen des brevets

(11) Veröffentlichungsnummer: **0 178 444**
**B1**

(12) # EUROPÄISCHE PATENTSCHRIFT

(45) Veröffentlichungstag der Patentschrift: **08.11.89**

(51) Int. Cl.⁴: **C 07 D 251/52**, C 08 K 5/36

(21) Anmeldenummer: **85111215.1**

(22) Anmeldetag: **05.09.85**

(54) **Bis-(2-Ethylamino-4-diethylamino-s-triazin-6-yl)tetrasulfid, Verfahren zur Herstellung, Verwendung und sie enthaltende vulkanisierbare Mischungen.**

(30) Priorität: **19.10.84 DE 3438290**

(43) Veröffentlichungstag der Anmeldung: **23.04.86 Patentblatt 86/17**

(45) Bekanntmachung des Hinweises auf die Patenterteilung: **08.11.89 Patentblatt 89/45**

(84) Benannte Vertragsstaaten: **AT BE CH DE FR GB IT LI LU NL SE**

(56) Entgegenhaltungen:
**EP-A- 0 038 459**
**DE-A- 2 848 559**
**GB-A- 3 923 724**

(73) Patentinhaber: **Degussa Aktiengesellschaft, Weissfrauenstrasse 9, D-6000 Frankfurt am Main 1 (DE)**

(72) Erfinder: **Schwarze, Werner, Dr., Tiroler Strasse 101, D-6000 Frankfurt am Main (DE)**
Erfinder: **Wolff, Siegfried, Weiherstrasse 28, D-5303 Bornheim-Merten (DE)**
Erfinder: **Remmel, Hans, Altenmittlauer Strasse 44, D-6463 Freigericht (DE)**
Erfinder: **Lambertz, Horst, Kreuzstrasse 37, D-5030 Hürth (DE)**

**EP 0 178 444 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft das «Bis- (2-Ethylamino-4-diethylamino-s-triazin- 6-yl) tetrasulfid (V 480), ein Verfahren zur Herstellung, ihre Verwendung und sie enthaltende vulkanisierbare Mischungen. Das entsprechende Disulfid ist aus der DE-PS 1 669 954 bekannt. Man kann es beispielsweise aus dem entsprechenden Monomercaptotriazin durch Oxidation mit Jod oder Wasserstoffperoxid herstellen. Die so gewonnene Verbindung wird als Vulkanisationsbeschleuniger in Kautschukmischungen eingesetzt.

Aus der EP-A- 0038 459 ist ein Vulkanisationssystem bekannt, das neben Schwefel oder einem Schwefelspender einen Beschleuniger der Mercapto- bzw. Sulfenamid-Gruppe, einen weiteren konventionellen Zusatzbeschleuniger und eine Verbindung, die einen Thiophenylrest trägt, enthält.

Aufgabe der Erfindung ist eine Verbindung, die den Vulkanisaten noch bessere Eigenschaften verleiht, und ein Verfahren zu deren Herstellung.

Gegenstand der Erfindung ist das Bis- (2-Ethylamino- 4-diethylamino-s-triazin- 6-yl)-tetrasulfid (V 480) und ein Verfahren zu seiner Herstellung, das dadurch gekennzeichnet ist, dass man eine wässrige, alkalische Lösung von 2-Ethylamino-4-diethylamino-6-mercaptotriazin in einem 2-Phasensystem mit einer Lösung von $S_2Cl_2$ in einem inerten organischen Lösungsmittel bei Temperaturen $< +10\,°C$ umsetzt, mit der Massgabe, dass das Lösungsmittel das entstehende Tetrasulfid nicht oder nur wenig löst.

Vorteilhaft stellt man eine alkalische wässrige Lösung des Mercaptotriazins her, die Alkaliionen und Mercaptotriazinmoleküle in äquimolaren Mengen enthält.

Bevorzugt wird jedoch eine Menge an Alkali, insbesondere Natriumhydroxid, die um 5 bis 10% höher liegt.

Diese Lösung vermengt man mit einem organischen, besonders aliphatischen oder cycloaliphatischen Lösungsmittel, insbesondere Benzine, Petroläther oder Cyclohexan, so dass sich ein 2-Phasensystem ergibt, und gibt eine Lösung von $S_2Cl_2$ bevorzugt in dem Lösungsmittel zu, das auch zuvor mit der Lösung des Mercaptotriazins vermengt wurde. Die Temperatur soll dabei unter 10 °C, bevorzugt unter 5 °C liegen.

$S_2Cl_2$ wird in äquimolaren Mengen, bevorzugt im Verhältnis 1,01:1 bis 1,1:1, unter starkem Rühren zur Reaktion gebracht.

Unter den gegebenen Bedingungen wirkt das $S_2Cl_2$ überraschenderweise ausschliesslich kondensierend.

Das ausfallende Produkt wird mit Hilfe allgemein bekannter Massnahmen abgetrennt und vorteilhaft bei 40–45 °C unter Vakuum getrocknet.

Gegenstand der Erfindung sind ebenso die Verwendung von V 480 in vulkanisierbaren Kautschukmischungen und die entsprechenden V 480 enthaltenden Mischungen selbst.

Die erfindungsgemässe Verbindung V 480 zeigt sich bei ihrer Verwendung als Vernetzer bzw. Vulkanisationsbeschleuniger den Standardverbindungen ebenso wie auch dem Disulfid V 143 als deutlich überlegen.

Der kautschukverarbeitenden Industrie steht eine umfangreiche Palette von Beschleunigern, vorzugsweise für die Schwefelvulkanisation, zur Verfügung, von denen die wichtigsten Klassen für all purpose-Kautschuke sind: Benzthiazolylsulfenamide, Bis-Benzthiazolyldisulfid und 2-Mercaptobenzthiazol sowie deren entsprechende Triazinderivate. Daneben gibt es eine Reihe spezieller Verbindungen wie Thiuramdisulfide und Peroxide, die auch ohne weitere Zusatzstoffe wie Schwefel als Vernetzer wirken, aber auch oft in Kombination mit Schwefel verwendet werden.

Die auch mengenmässig grösste Bedeutung in der praktischen Anwendung, insbesondere zur Vulkanisation von all purpose-Kautschuken, kommt heute den Benzthiazolylsulfenamiden zu.

Ein wesentlicher Nachteil der eben genannten Vulkanisationsbeschleuniger, insbesondere der Sulfenamide, ist ihre mit steigender Vulkanisationstemperatur stark ansteigende Neigung zur Reversion beim Überheizen der Vulkanisate, besonders bei Verwendung ohnehin reversionsanfälliger Kautschukarten wie NR und Polyisopren. Mit steigender Temperatur nimmt die Reversionsgeschwindigkeit so stark zu, dass es einerseits zu einer drastischen Absenkung der Vernetzungsdichte bei optimaler Vulkanisation kommt und andererseits zu einem starken Abfall der optimalen Vernetzungsdichte bei einer oft unvermeidbaren Übervulkanisation. Gleiches trifft, wenn auch abgeschwächt, auf die übrigen Beschleuniger der Benzthiazolklasse zu.

Diese Nachteile der Benzthiazolbeschleuniger schränken ihre Verwendbarkeit mit steigender Vulkanisationstemperatur ein und setzen Grenzen hinsichtlich der Bestrebungen der kautschukverarbeitenden Industrie zur Produktivitätssteigerung durch Verwendung höherer Vulkanisationstemperaturen.

Ein weiterer heute nicht zu vernachlässigender Nachteil, insbesondere der Sulfenamide, liegt darin, dass während des Vulkanisationsprozesses freie Amine entstehen, die – soweit sie nitrosierbar sind – zur Bildung toxischer Nitrosamine führen können, was zukünftig eine Beschränkung ihrer Einsatzmöglichkeiten durch den Gesetzgeber erwarten lässt.

Überraschenderweise erweist sich V 480 sowohl hinsichtlich seiner Verwendung als Vernetzer als auch als Vulkanisationsbeschleuniger bei der Schwefelvulkanisation als eine Verbindung, die den damit hergestellten Vulkanisaten auch bei hohen Vulkanisationstemperaturen ausserordentlich hohe Reversionsbeständigkeit verleiht, sie daher für die Verwendung bei der Hochtemperaturvulkanisation prädestiniert und damit Produktivitätssteigerungen ermöglicht.

Die Verwendung von V480 umfasst die nach dem Stand der Technik bekannten Kautschukmischungen auf der Basis von Naturkautschuk (NR), Isoprenkautschuk (IR), Styrol-Butadienkautschuk (SBR), Isobutylen-Isoprenkautschuk

(IIR), Ethylen-Propylen-Terpolymer (EPDM), Nitrilkautschuk (NBR), halogenhaltige Kautschuke und insbesondere Naturkautschuk, der bis zu 75% epoxidiert ist (ENR), sowie deren Gemische. Wesentlich ist das Vorhandensein von Doppelbindungen. Besondere Bedeutung hat die Verwendung von V480 für die reversionsanfälligen Isopren- und Naturkautschuke, sowie deren Verschnitte mit anderen Kautschuken, insbesondere epoxidiertem Naturkautschuk. V480 wird in Schwefel-haltigen Kautschukmischungen in einer Menge von 0,3 bis 15, bevorzugt 0,3 bis 5 Gew.-Teilen, auf 100 Teile Kautschuk eingesetzt.

In Schwefel-freien Kautschukmischungen verwendet man 0,3 bis 10, bevorzugt 0,3 bis 5 Gew.-Teile V480 pro 100 Teile Kautschuk.

Ansonsten enthalten die Kautschukmischungen die

- üblichen Verstärkungssysteme, d.h. Furnace-Russe, Channelrusse, Flammrusse, Thermalrusse, Acetylenrusse, Lichtbogenrusse, CK-Russe usw. sowie synthetische Füllstoffe wie Kieselsäuren, Silikate, Aluminiumoxidhydrate, Calciumcarbonate und natürliche Füllstoffe wie Clays, Kieselkreiden, Kreiden, Talke usw. und deren Verschnitte in Mengen von 5 bis 300 Teilen je 100 Teilen Kautschuk,
- ZnO und Stearinsäure als Promotoren der Vulkanisation in Mengen von 2 bis 5 Teilen,
- üblicherweise verwendeten Alterungs-, Ozon-, Ermüdungsschutzmitteln wie z.B. IPPD, TMQ sowie auch Wachsen als Lichtschutzmittel und deren Verschnitte,
- beliebigen Weichmachern wie z.B. aromatische, naphthenische, paraffinische, synthetische Weichmacher und deren Verschnitte,

gegebenenfalls Verzögerer wie z.B. N-Cyclohexylthiophthalimid, (N-trichlormethylthio-phenylsulfonyl)-Benzol und deren Verschnitte,
gegebenenfalls Silane wie z.B. Bis-(3-triäthoxisilylpropyl)-tetrasulfid, γ-Chlorpropyltriäthoxisilan, γ-Mercaptopropyltrimethoxisilan,

$$[(C_2H_5O)_3Si(CH_2)_2- \quad CH_3\ ]_2\,[S_{\sim 3}]$$

und deren Verschnitte, in einer Menge von 0,1 bis 20, bevorzugt 1 bis 10 Teilen je 100 Teile Füllstoff,
gegebenenfalls Schwefel in einer Menge von 0,5 bis 4 Teilen je 100 Teile Kautschuk
gegebenenfalls zusätzliche in der Kautschukindustrie sonst üblichen Beschleuniger als Zweitbeschleuniger, insbesondere Vulkalent E und V480 im Molverhältnis 1:1 bei einer Schwefeldosierung von 0,2 bis 4 Teilen, bevorzugt 0,6–1,8 Teilen,
gegebenenfalls zustäzlichen Schwefelspendern, gegebenenfalls Farbstoffen und Verarbeitungshilfsmitteln.

Der Anwendungsbereich erstreckt sich auf Kautschukmischungen, wie sie üblicherweise im Reifenbau verwendet werden, auf technische Artikel, wie z.B. Mischungen für Fördergurte, Keilriemen, Formartikel, Schläuche mit und ohne Einlagen, Walzengummierungen, Auskleidungen, Spritzprofile, Freihandartikel, Folien, Schuhsohlen und Oberteile, Kabel, Vollgummireifen und deren Vulkanisate.

Beispiel 1

Man löst 454 g 2-Diethylamino-4-ethylamino-6-mercaptotriazin in Natronlauge, die man aus 84 g NaOH+1,5 ltr. $H_2O$ hergestellt hat.

Die Lösung gibt man in einen 4 ltr. 3 Tubenkolben, dann gibt man 1,5 ltr. Leichtbenzin (Kp 80–110°) hinzu und kühlt die Mischung unter starkem Rühren auf 0 °C ab.

Nun lässt man innerhalb von 20 min. eine Lösung von 137 g $S_2Cl_2$ in 100 ml Benzin einlaufen, wobei man darauf achtet, dass die Temperatur + 5 °C nicht überschreitet.

Das Tetrasulfid fällt sofort aus. Am Ende der Reaktion wird 5 min. nachgerührt, anschliessend abgenutscht und gewaschen.

Das schneeweisse feine Pulver wird im Vakuum/12 Torr bei 40–45 °C getrocknet.

Menge: 499,5 g entsprechend 97,1% d.Th., F. 149–150 °C.

Analyse:

Bis-(2-Ethylamino -4-diethylamino-s-triazin-6-yl)-tetrasulfid, Mol-Gew. 516, $C_{18}H_{32}N_{10}S_4$

| | | | | |
|---|---|---|---|---|
| ber. | C 41,9 | H 6,2 | N 27,1 | S 24,8 |
| gef. | 41,8 | 6,5 | 26,8 | 24,8 |

Prüfungsnormen

Die physikalischen Prüfungen wurden bei Raumtemperatur nach folgenden Normvorschriften ausgeführt:

| | | gemessen in |
|---|---|---|
| Zugfestigkeit, Bruchdehnung und Spannungswert an 6 mm starken Ringen | DIN 53 504 | MPa |
| Weiterreisswiderstand | DIN 53 507 | N/mm |
| Stosselastizität | DIN 53 512 | % |
| Shore-A-Härte | DIN 53 505 | – |
| Mooney-Prüfung, ML 4 | DIN 53 524 | – |
| Goodrich Flexometer (Bestimmung der Wärmebildung = Heat build-up. ΔT) | ASTM D 623-62 | °C |
| Firestone-Ball Rebound | AD 20245 | |
| $D_{max}-D_{(max+60')}$ | DIN 53 529 | % |
| $D_{max}-D_{min}$ | DE-PS-2 848 559 | |

In den Anwendungs-Beispielen werden folgende Namen und Abkürzungen benutzt, deren Bedeutung im folgenden angegeben wird.

| | |
|---|---|
| RSS: | Ribbed Smoked Sheet (Naturkautschuk) |
| Corax®N 220: | Russ, Oberfläche (BET) 120 $m^2/g$ (Degussa) |
| Naftolen®ZD: | Weichmacher aus Kohlenwasserstoffen |
| Ingralen®450: | Weichmacher aus aromatischen Kohlenwasserstoffen |
| Ingroplast®NS: | Weichmacher aus naphtenischen Kohlenwasserstoffen |
| Vulkanox®4010 NA: | N-Isopropyl-N'-phenyl-p-phenylendiamin |
| Vulkanox®HS: | Poly-2,2,4-trimethyl-1,2-dihydrochinolin |
| Mesamoll:® | Alkylsulfonsäureester von Phenol und Kresol |
| Protektor®G35: | Ozonschutzwachs |
| Vulkacit®MOZ: | N-Morpholin-2-benzthiazolsulphenamid |
| Vulcacit®Mercapto: | 2-Mercaptobenzthiazol |
| Vulcacit®Thiuram: | Tetramethyl-thiurammonosulfid |
| Vulcazit®CZ: | N-Cyclohexyl-2-benzothiazolsulphenamid |
| Vulcalent®E: | (N-trichlormethylthiophenylsulfonyl)-benzol |
| PVI: | N-Cyclohexylthiophtalimid |
| Ultrasil®VN3: | gefällte Kieselsäure (Degussa) |
| Gran. | Granulat |
| V143: | Bis-(2-Ethylamino-4-diethylamino-s-triazin-6-yl)-disulfid |

Beispiel 2

Reversionsstabilität mit V 480 als Vernetzer (Russ als Füllstoff)

| | 1 | 2 | 3 |
|---|---|---|---|
| RSS 1, ML 4=67 | 100 | 100 | 100 |
| CORAX N 220 | 50 | 50 | 50 |
| ZnO RS | 5 | 5 | 5 |
| Stearinsäure | 2 | 2 | 2 |
| Naftolen ZD | 3 | 3 | 3 |
| Vulkanox 4010 NA | 2,5 | 2,5 | 2,5 |
| Vulkanox HS | 1,5 | 1,5 | 1,5 |
| Protektor G 35 | 1 | 1 | 1 |
| Vulkacit MOZ | 1,43 | – | – |
| V 143 | – | 1,29 | – |
| PVI | – | 0,4 | – |
| V 480 | – | – | 4 |
| Schwefel | 1,5 | 1,5 | – |
| $\frac{D_{max} - D_{(max+60')}}{D_{max} - D_{min}}$ (% | | | |
| 170 °C | 30,0 | 8,5 | 2,3 |

Dieses Beispiel zeigt, dass bei Verwendung von V 480 ohne Schwefel Reversionsstabilität erreicht wird. Als Referenzsysteme werden in Mischung 1 MOZ in einer sogenannten semi-efficient-Dosierung, die nach dem Stand der Technik als sehr gut beurteilt wird, verwendet und in Probe 2 der ohnehin schon sehr reversionsstabile Beschleuniger V 143

Beispiel 3

Temperaturabhängigkeit des Reversionsverhaltens bei Verwendung von V 480 (Russ/Kieselsäure als Füllstoffe)

| | 4 | 5 | 6 |
|---|---|---|---|
| RSS 1, ML 4=67 | 100 | 100 | 100 |
| CORAX N 220 | 25 | 25 | 25 |
| Ultrasil VN 3 Gran. | 25 | 25 | 25 |
| ZnO RS | 5 | 5 | 5 |
| Stearinsäure | 2 | 2 | 2 |
| Naftolen ZD | 3 | 3 | 3 |
| Vulkanox 4010 NA | 2,5 | 2,5 | 2,5 |
| Vulkanox HS | 1,5 | 1,5 | 1,5 |
| Protektor G 35 | 1,5 | 1,5 | 1,5 |
| V 480 | – | – | 3 |
| Vulkacit MOZ | 1,43 | – | – |
| V 143 | – | 1,29 | – |
| Schwefel | 1,5 | 1,5 | – |
| $\frac{D_{max} - D_{(max+60')}}{D_{max} - D_{min}}$ (%) | | | |
| 145 °C | 22,4 | 11,3 | 0 |
| 160 °C | 38,8 | 20,9 | 0 |
| 170 °C | 47,4 | 30,3 | 1,9 |
| 180 °C | 52,6 | 38,7 | 4,6 |

Mischungen, in denen Russ zum Teil durch Kieselsäure ersetzt ist, sind besonders reversionsanfällig. Mischung 6 zeigt, dass V 480 als Vernetzer verwendet, d.h. ohne Schwefel, dem Vulkanisat auch bei höchsten Vulkanisationstemperaturen äusserste Reversionsbeständigkeit verleiht.

Beispiel 4

Vulkanisatstabilität bei Überheizung bei 170 °C und Verwendung von V480

| | 7 | 8 | 9 |
|---|---|---|---|
| RSS 1, ML 4=67 | 100 | 100 | 100 |
| CORAX N 220 | 25 | 25 | 25 |
| Ultrasil VN 3 Gran. | 25 | 25 | 25 |
| ZnO RS | 5 | 5 | 5 |
| Stearinsäure | 2 | 2 | 2 |
| Naftolen ZD | 3 | 3 | 3 |
| Vulkanox 4010 NA | 2,5 | 2,5 | 2,5 |
| Vulkanox HS | 1,5 | 1,5 | 1,5 |
| Protektor G 35 | 1 | 1 | 1 |
| Vulkacit MOZ | 1,43 | – | – |
| V 143 | – | 1,29 | – |
| V 480 | – | – | 3 |
| Schwefel | 1,5 | 1,5 | – |
| $\frac{D_{max} - D_{(max+60')}}{D_{max} - D_{min}}$ (%) | | | |
| 170 °C | 44,7 | 28,7 | 2,6 |
| Vulkanisationszeit bei 170 °C | | *) $t_{95\%}$ $t_{95\%,50'}$ | |
| Zugfestigkeit | 17,2 | 16,0 | 19,3 |
| | 12,5 | 11,2 | 19,7 |
| Spannungswert 300% | 5,1 | 3,7 | 5,5 |
| | 3,3 | 2,8 | 5,3 |
| Weiterreisswiderstand | 32 | 16 | 29 |
| | 6 | 5 | 28 |

| | | | |
|---|---|---|---|
| Firestone-Ball | 54,9 | 52,8 | 53,5 |
| Rebound | 51,3 | 51,7 | 53,2 |

Dieses Beispiel zeigt, dass mit steigender Reversion bei Überheizung, nämlich um 50'/170 °C, ein starker Abfall der physikalischen Vulkanisatdaten eintritt. Besonders deutlich ist dies zu sehen bei Mischung 7 in der Zerreissfestigkeit und im 300%-Spannungswert sowie im Weiterreisswiderstand, während dagegen Mischung 9 bei Überheizung praktisch unverändert die physikalischen Daten beibehält.

Auch hier wird V 480 gegen ein semi-EV-System verglichen, das nach dem Stand der Technik schon als reversionsfest angesehen wird.

*) $t_{95\%}$ bedeutet, dass 95% der Vulkanisationsmittel umgesetzt worden sind; $t_{95\%+50'}$ bedeutet, dass anschliessend noch 50 Minuten geheizt wird.

Beispiel 5

Reversionsbeständigkeit bei Verwendung von V 480 als Beschleuniger bei einer Vulkanisationstemperatur 170 °C

| | 10 | 11 |
|---|---|---|
| RSS 1, ML 4=67 | 100 | 100 |
| CORAX N 220 | 50 | 50 |
| ZnO RS | 5 | 5 |
| Stearinsäure | 2 | 2 |
| Naftolen ZD | 3 | 3 |
| Vulkanox 4010 NA | 2,5 | 2,5 |
| Vulkanox HS | 1,5 | 1,5 |
| Protektor G 35 | 1 | 1 |
| Vulkacit MOZ | – | 1,43 |
| V 480 | 1,5 | – |
| Schwefel | 0,8 | 1,5 |
| $\frac{D_{max} - D_{(max+60')}}{D_{max} - D_{min}}$ (%) | 0,8 | 29,2 |
| Zugfestigkeit | 22,6 | 24,3 |
| Spannungswert 300% | 11,0 | 10,4 |
| Bruchdehnung | 480 | 530 |
| Firestone-Ball Rebound | 46,5 | 45,9 |
| Shore-Härte | 62 | 62 |

Beispiel 5 zeigt, dass die Kombination von 1,5 Tln. V 480 mit 0,8 Tln. Schwefel immer noch im Vergleich zum entsprechenden Sulfenamid bei 170 °C völlig reversionsfest bleibt und dass mit dieser Kombination bei $t_{95\%}$ praktisch gleiches Datenniveau eingestellt wird.

Beispiel 6

Einfluss der Schwefeldosierung auf die V 480-Beschleunigung (Vulkanisationstemperatur: 170 °C)

| | 12 | 13 | 14 | 15 | 16 | 17 |
|---|---|---|---|---|---|---|
| RSS 1, ML 4=67 | 100 | 100 | 100 | 100 | 100 | 100 |
| CORAX N 220 | 50 | 50 | 50 | 50 | 50 | 50 |
| ZnO RS | 5 | 5 | 5 | 5 | 5 | 5 |
| Stearinsäure | 2 | 2 | 2 | 2 | 2 | 2 |
| Naftolen ZD | 3 | 3 | 3 | 3 | 3 | 3 |
| Vulkanox 4010 NA | 2,5 | 2,5 | 2,5 | 2,5 | 2,5 | 2,5 |
| Vulkanox HS | 1,5 | 1,5 | 1,5 | 1,5 | 1,5 | 1,5 |
| Protektor G 35 | 1 | 1 | 1 | 1 | 1 | 1 |
| Vulkacit MOZ | 1,43 | – | – | – | – | – |
| V 143 | – | 1,29 | – | – | – | – |
| PVI | – | 0,4 | – | – | – | – |
| V 480 | – | – | 1,5 | 1,5 | 1,5 | 1,5 |
| Schwefel | 1,5 | 1,5 | 0,8 | 1 | 1,2 | 1,4 |
| $\frac{D_{max} - D_{(max+60')}}{D_{max} - D_{min}}$ (%) | 26,8 | 6,9 | 2,4 | 3,4 | 4,1 | 4,6 |
| $t_{10\%}$ | 3,8 | 4,2 | 3,1 | 2,9 | 2,9 | 2,8 |
| $t_{80}-t_{20\%}$ | 1,4 | 1,4 | 1,6 | 1,6 | 1,5 | 1,5 |
| Vulkanisatdaten bei $t_{95\%}$ | | | | | | |
| Spannungswert 300% | 11,5 | 12,1 | 11,4 | 12,1 | 12,5 | 13,1 |
| Shore-Härte | 63 | 66 | 63 | 63 | 64 | 65 |

Beispiel 6 zeigt, dass eine Steigerung des Schwefelgehaltes über 0,8 hinaus möglich ist und zu Modulsteigerungen führt, ohne dass die Reversion sehr stark ansteigt. Allerdings hat die Anhebung des Schwefelgehaltes eine geringfügige Verkürzung des Scorchverhaltens zur Folge. Dieser kann durch Verwendung von Vulkalent E aufgehoben werden (s. Beispiel 7).

Beispiel 7

Wirkung üblicher Verzögerer auf die Anvulkanisationszeit und Reversion bei Einsatz von V 480

| | 18 | 19 | 20 | 21 |
|---|---|---|---|---|
| RSS 1, ML (1+4)=67 | 100 | 100 | 100 | 100 |
| CORAX N 220 | 50 | 50 | 50 | 50 |
| ZnO RS | 5 | 5 | 5 | 5 |
| Stearinsäure | 2 | 2 | 2 | 2 |
| Naftolen ZD | 3 | 3 | 3 | 3 |
| Vulkanox 4010 NA | 2,5 | 2,5 | 2,5 | 2,5 |
| Vulkanox HS | 1,5 | 1,5 | 1,5 | 1,5 |
| Protektor G 35 | 1 | 1 | 1 | 1 |
| Vulkacit MOZ | 1,43 | – | – | – |
| V 480 | – | 1,5 | 1,5 | 1,5 |
| Schwefel | 1,5 | 0,8 | 0,8 | 0,8 |
| PVI | – | – | 1,2 | – |
| Vulkalent E | – | – | 1,2 | – |
| Scorchzeit 130 °C min (Anstieg 2 Skalenteile) | 21,5 | 8,0 | 17,5 | 21,0 |
| Scorch bei 170 °C ($t_{10\%}$) | 3,8 | 2,8 | 3,8 | 4,1 |
| Spannungswert 300% | 10,6 | 11,0 | 8,8 | 13,7 |

Beispiel 8

Scorchverlängerung und Modulerhöhung von V 480/Vulcalent E-Kombination

| | 22 | 23 | 24 | 25 | 26 |
|---|---|---|---|---|---|
| RSS 1, ML (1+4)=67 | 100 | 100 | 100 | 100 | 100 |
| CORAX N 220 | 50 | 50 | 50 | 50 | 50 |
| ZnO RS | 5 | 5 | 5 | 5 | 5 |
| Stearinsäure | 2 | 2 | 2 | 2 | 2 |
| Naftolen ZD | 3 | 3 | 3 | 3 | 3 |
| Vulkanox 4010 NA | 2,5 | 2,5 | 2,5 | 2,5 | 2,5 |
| Vulkanox HS | 1,5 | 1,5 | 1,5 | 1,5 | 1,5 |
| Protektor G 35 | 1 | 1 | 1 | 1 | 1 |
| Vulkacit MOZ | 1,43 | – | – | – | – |
| V 480 | – | 1,5 | 1,5 | 1,5 | 1,5 |
| Vulkalent E | – | – | 0,4 | 0,8 | 1,2 |
| Schwefel | 1,5 | 0,8 | 0,8 | 0,8 | 0,8 |
| Scorchzeit 130 °C, min. (Anstieg 2 Skalenteile) | 21,5 | 8,0 | 12,5 | 16,7 | 21,0 |
| Scorchzeit 170 °C ($t_{10\%}$), min | 3,8 | 2,8 | 3,1 | 3,7 | 4,1 |
| Spannungswert 300% | 10,6 | 11,0 | 11,8 | 12,7 | 13,7 |

Beispiel 9

Verlängerung der Anvulkanisationszeit durch Vulkalent E bei der V 480-Vulkanisation

| | 27 | 28 | 29 | 30 |
|---|---|---|---|---|
| RSS 1, ML (1+4) = 67 | 100 | 100 | 100 | 100 |
| CORAX N 220 | 25 | 25 | 25 | 25 |
| Ultrasil VN3 Gran. | 25 | 25 | 25 | 25 |
| ZnO RS | 5 | 5 | 5 | 5 |
| Stearinsäure | 2 | 2 | 2 | 2 |
| Naftolen ZD | 3 | 3 | 3 | 3 |
| Vulkanox 4010 NA | 2,5 | 2,5 | 2,5 | 2,5 |
| Vulkanox HS | 1,5 | 1,5 | 1,5 | 1,5 |
| Protektor G 35 | 1 | 1 | 1 | 1 |
| Vulkacit MOZ | 1,43 | – | – | – |
| V 480 | – | 3 | 3 | 1,5 |
| PVI | – | – | 1,2 | – |
| Vulkalent E | – | – | – | 1,2 |
| Schwefel | 1,5 | 0,8 | 0,8 | 0,8 |
| Scorchzeit 130 °C, min. (Anstieg 2 Skalenteile) | 29,5 | 16,1 | 28,5 | 30,0 |
| Scorchzeit 170 °C | 4,5 | 3,6 | 4,2 | 4,7 |
| Spannungswert 300% | 5,3 | 6,4 | 6,4 | 8,6 |

Beispiel 9 zeigt im Falle eines Russ/Kieselsäure-Verschnittes die Wirksamkeit des Verzögerers Vulkalent E. Bei einer Dosierung von 1,5 Tln. V 480, 0,8 Tln. Schwefel und 1,2 Tln. Vulkalent E werden MOZ-Anvulkanisationszeiten ohne weiteres erreicht. Auch bei Einsatz von Verzögerern wird das Reversionsverhalten der V 480-Vulkanisation nicht negativ beeinflusst, ebenso wenig wie die physikalischen Daten des Vulkanisates.

Beispiel 10

V 480 als Beschleuniger in SBR

| | 31 | 32 | 33 |
|---|---|---|---|
| SBR 1712 | 137,5 | 137,5 | 137,5 |
| CORAX N 339 | 60 | 60 | 60 |
| ZnO RS | 3 | 3 | 3 |
| Stearinsäure | 2 | 2 | 2 |
| Protektor G 35 | 1 | 1 | 1 |
| Vulkanox 4010 NA | 1,5 | 1,5 | 1,5 |
| Vulkacit D | 0,5 | 0,5 | – |
| Vulkacit CZ | 1,45 | – | – |
| V 480 | – | 1,5 | 1,5 |
| Schwefel | 1,6 | 1,5 | 1,5 |
| $\frac{D_{max} - D_{(max+60')}}{D_{max} - D_{min}}$ (%) | 10,5 | 7,3 | 8,3 |
| bei 165 °C | | | |
| Zerreissfestigkeit | 20 | 19,2 | 23,1 |
| Spannungswert 300% | 10,1 | 11,4 | 10,9 |
| Bruchdehnung | 480 | 430 | 460 |
| Shore-Härte | 63 | 65 | 64 |

Beispiel 10 zeigt, dass V 480 auch in ohnehin schon reversionsfesteren SBR-Mischungen einen positiven Einfluss auf die Reversionsbeständigkeit ausübt.

Beispiel 11

Reversionsfestigkeit von SBR-Vulkanisaten mit V 480

| | 33 | 34 |
|---|---|---|
| SBR 1500 | 100 | 100 |
| CORAX N 220 | 50 | 50 |
| ZnO RS | 5 | 5 |
| Stearinsäure | 2 | 2 |
| Naftolen ZD | 3 | 3 |
| Vulkanox 4010 NA | 2,5 | 2,5 |
| Vulkanox HS | 1,5 | 1,5 |
| Protektor G 35 | 1 | 1 |
| Vulkacit CZ | 1,5 | – |
| V 480 | – | 1 |
| Schwefel | 1,8 | 1,8 |
| $\frac{D_{max} - D_{(max+60')}}{D_{max} - D_{min\ (170°C)}}$ (%) | 12,1 | 9,1 |
| Vulkanisatdaten bei $t_{95\%}$: | | |
| Zerreissfestigkeit | 20,2 | 21,8 |
| Spannungswert 300% | 10,6 | 11,1 |
| Bruchdehnung | 450 | 460 |
| Weiterreisswiderstand | 13 | 14 |
| Shore-Härte | 63 | 64 |

Auch dieses Beispiel zeigt, dass V 480 im ohnehin schon wenig reversionsanfälligen SBR 1500 das Reversionsverhalten noch einmal verbessert.

Beispiel 12

V 480 in Perbunan (Nitrilkautschuk)

| | | |
|---|---|---|
| Perbunan N 3307 NS | 100 | 100 |
| CORAX N 220 | 50 | 50 |
| ZnO RS | 5 | 5 |
| Stearinsäure | 1 | 1 |
| Ingralen 450 | 5 | 5 |
| Mesamoll | 10 | 10 |
| Vulkacit CZ | 1,3 | – |
| V 480 | – | 1,5 |
| Schwefel | 1,8 | 1,8 |
| $\frac{D_{max} - D_{(max+60')}}{D_{max} - D_{min}}$ (%) | 9,5 | 6,9 |
| bei 170 °C | | |
| Vulkanisatdaten: | | |
| Zerreissfestigkeit | 19,5 | 18,8 |
| Spannungswert 300% | 9,2 | 11,3 |
| Bruchdehnung | 480 | 380 |
| Shore-Härte | 64 | 65 |

Wie das Beispiel zeigt, bringt der Einsatz von V 480 anstelle eines Sulfenamides in Perbunan weitere Vorteile hinsichtlich der Reversionsbeständigkeit.

Beispiel 13

V 480 in EPDM

| | | |
|---|---|---|
| Buna AP 541 | 100 | 100 |
| CORAX N 220 | 50 | 50 |
| ZnO RS | 5 | 5 |
| Stearinsäure | 1 | 1 |
| Ingraplast NS | 10 | 10 |
| Vulkacit Thiuram | 1 | – |
| Vulkacit Mercapto | 0,5 | – |
| V 480 | – | 2,5 |
| Schwefel | 1 | 1 |
| $\frac{D_{max} - D_{(max+60')}}{D_{max} - D_{min}}$ (%) | 3,3 | 0 |
| bei 170 °C | | |
| Vulkanisatdaten: | | |
| Zugfestigkeit | 16,0 | 16,0 |
| Spannungswert 300% | 14,4 | 14,0 |
| Bruchdehnung | 320 | 350 |
| Shore-Härte | 72 | 69 |

Auch für EPDM ergibt sich durch Einsatz von V 480 bei gleicher Einstellung der Vulkanisatdaten noch die Möglichkeit zur weiteren Heraufsetzung der Reversionsbeständigkeit.

Beispiel 14

Gleichzeitige Verwendung von V 480 und Si 69

| | | |
|---|---|---|
| RSS 1, ML 4=67 | 100 | 100 |
| CORAX N 220 | 50 | 50 |
| ZnO RS | 5 | 5 |
| Stearinsäure | 2 | 2 |
| Naftolen ZD | 3 | 3 |
| Vulkanox 4010 NA | 2,5 | 2,5 |
| Vulkanox HS | 1,5 | 1,5 |
| Protektor G 35 | 1 | 1 |
| Vulkacit MOZ | 1,43 | – |
| V 480 | – | 1,5 |
| Si 69 | – | 1,5 |
| Schwefel | 1,5 | 0,4 |
| $\frac{D_{max} - D_{(max+60')}}{D_{max} - D_{min}}$ (%) | 29,7 | 0 |
| bei 170 °C | | |
| Vulkanisatdaten: | | |
| Zerreissfestigkeit | 25,1 | 22,0 |
| Spannungswert 300% | 10,2 | 10,8 |
| Firestone-Ball Rebound | 45,2 | 44,2 |
| Shore-Härte | 63 | 62 |
| Goodrich-Flexometer | | |
| delta T Center °C | 159 | 136 |

Ersetzt man einen Teil des Schwefels (von 0,8 Tln.) durch Schwefelspender wie beispielsweise polysulfidische Silane, so ergeben sich ebenfalls ausserordentlich reversionsfeste Naturkautschukmischungen, wie obiges Beispiel zeigt. Darüberhinaus tritt eine ungewöhnliche Absenkung der Wärmebildung ein.

Beispiel 15

V 480 – Vernetzung von epoxidiertem Naturkautschuk bei Verwendung von Russ und Kieselsäure als Füllstoff

| | 1 | 2 |
|---|---|---|
| ENR 50 | 100 | 100 |
| CORAX N 330 | 25 | 25 |
| Ultrasil VN 3 Gran. | 25 | 25 |
| ZnO RS | 5 | 5 |
| Stearinsäure | 2 | 2 |
| Vulkanox HS | 2 | 2 |
| V 480 | – | 3 |
| Vulkacit MOZ | 2,4 | – |
| Vulkacit Thiuram | 1,6 | – |
| Schwefel | 0,3 | 0,3 |
| Zugfestigkeit | 15,1 | 15,6 |
| Feststoffdispersion+ | | |
| Spannungswert 100% (MPa) | 8,4 | 11,0 |
| Weiterreisswiderstand DIN 53 507 (N/mm) | 8 | 8 |
| Shore-A-Härte DIN 53 505 23 °C | 82 | 89 |

Beispiel 16

V 480 – Vernetzung von Epoxidiertem Naturkautschuk bei Russfüllung

| | 1 | 2 |
|---|---|---|
| ENR | 100 | 100 |
| CORAX N 220 | 50 | 50 |
| ZnO RS | 5 | 5 |
| Stearinsäure | 2 | 2 |
| Vulkanox HS | 2 | 2 |
| V 480 | – | 4 |
| Vulkacit MOZ | 2,4 | – |
| Vulkacit Thiuram | 1,6 | – |
| Schwefel | 0,3 | 0,3 |
| Zufgestigkeit DIN 53 504 Ring 1 (MPa) | 18,7 | 27,0 |
| Spannungswert 300% (MPa) | 18,0 | 19,0 |
| Weiterreisswiderstand DIN 53 507 (N/mm) | 12 | 12 |
| Shore-A-Härte DIN 53 505 23 °C | 75 | 80 |

## Patentansprüche

1. Bis-(2-Ethylamino -4-diethylamino -s-triazin -6-yl)tetrasulfid

2. Verfahren zur Herstellung der Verbindung gemäss Anspruch 1, dadurch gekennzeichnet, dass man eine wässrige alkalische Lösung von 2-Ethylamino-4-diethylamino -6-mercaptotriazin in einem 2-Phasensystem mit einer Lösung von $S_2Cl_2$ in einem inerten, das entstehende Tetrasulfid nicht oder nur wenig lösenden, organischen Lösungsmittel bei Temperaturen $< +10\,°C$ umsetzt.

3. Verfahren gemäss Anspruch 2, dadurch gekennzeichnet, dass man mindestens eine Alkalimenge verwendet, die zur Bildung des Alkalimercaptids notwendig ist oder eine Menge, die 5–10% darüber liegt.

4. Verfahren gemäss den Ansprüchen 2 und 3, dadurch gekennzeichnet, dass man als Lösungsmittel Benzine, Petroläther oder Cyclohexan verwendet.

5. Verwendung der Verbindung gemäss Anspruch 1 als Beschleuniger in vulkanisierbaren, Füllstoffe, Schwefel und weitere übliche Bestandteile enthaltenden Mischungen auf der Basis eines oder mehrerer natürlicher und/oder synthetische Kautschuk(e)(s), die gegebenenfalls einen weiteren Beschleuniger und/oder Verzögerer und oder Organosilan enthalten.

6. Verwendung von 0,3 bis 10 Gew.-Teilen der Verbindung gemäss Anspruch 1 pro 100 Teile Kautschuk als Vernetzer in vulkanisierbaren, Schwefel ($S_8$)-freien, Füllstoffe und weitere übliche Bestandteile enthaltenden Mischungen auf der Basis eines oder mehrerer natürlicher und/oder synthetischer Kautschuk(e)(s), die gegebenenfalls einen weiteren Beschleuniger und/oder Verzögerer und/oder Organosilan enthalten.

7. Vulkanisierbare, Füllstoffe, Schwefel und weitere übliche Bestandteile enthaltende Mischungen auf der Basis eines oder mehrer natürlicher und/oder synthetischer Kautschuk(e)(s), dadurch gekennzeichnet, dass sie 0,3 bis 15 Teile der Verbindung gemäss Anspruch 1 enthalten.

8. Vulkanisierbare Mischung gemäss Anspruch 7, dadurch gekennzeichnet, dass sie Vulkalent E [(N-trichloromethylthiophenyl-sulphonyl)-benzol] und die Verbindung gemäss Anspruch 1 im Molverhältnis 1:1 bei einer Schwefeldosierung von 0,2 bis 4, vorzugsweise 0,6 bis 1,8 Teilen enthält.

9. Vulkanisierbare, Füllstoffe und weitere übliche Bestandteile enthaltende, Schwefel ($S_8$)-freie Mischungen auf der Basis eines oder mehrer natürlicher und/oder synthetischer Kautschuke, dadurch gekennzeichnet, dass sie 0,3 bis 10 Teile der Verbindung gemäss Anspruch 1 enthalten.

## Revendications

1. Tétrasulfure de Bis-(2-éthylamino -4-diéthylamino -s-triazinyl-6).

2. Procédé de préparation du composé selon la revendication 1, caractérisé en ce qu'on fait réagir une solution aqueuse alcaline de 2-éthyl-amino -4-diéthyl-amino -6-mercaptotriazine dans un système biphasé avec une solution de $S_2Cl_2$ dans un solvant organique interne, ne dissolvant peu ou pas le tétrasulfure formé à des températures $< +10\,°C$.

3. Procédé selon la revendication 2, caractérisé en ce qu'on utilise au moins une quantité d'alcali qui est nécessaire à la formation du mercaptide alcalin, ou une quantité qui lui est supérieure de 5–10%.

4. Procédé selon les revendications 2 et 3, caractérisé en ce qu'on utilise, comme solvant des essences, de l'éther de pétrole ou du cyclohexane.

5. Utilisation du composé selon la revendication 1 comme accélérateur dans des mélanges comprenant des charges, du soufre et d'autres composants usuels et vulcanisables, à base d'un ou plusieurs caoutchoucs naturels et/ou synthétiques, qui comprennent, le cas échéant, un autre accélérateur et/ou un retardateur et/ou un organosilane.

6. Utilisation de 0,3 à 10 parties en poids du composé selon la revendication 1, pour 100 parties de caoutchouc, comme réticulant dans des mélanges vulcanisables comprenant du soufre exempt de $S_8$, des charges et d'autres composants usuels, sur la base d'un ou plusieurs caoutchoucs naturels et/ou synthétiques, qui comprennent le cas échéant un autre accélérateur et/ou retardateur et/ou organosilane.

7. Mélanges vulcanisables comprenant des charges, du soufre et d'autres composants usuels, sur la base d'un ou plusieurs caoutchoucs naturels et/ou synthétiques, caractérisés en ce qu'ils comprennent de 0,3 à 15 parties du composé selon la revendication 1.

8. Mélange vulcanisable selon la revendication 7, caractérisés en ce qu'il contient du Vulkalent E[(N-trichlorométhylthiophènesulfonyl)-benzène] et le composé de la revendication 1 en rapport molaire 1:1 pour une dose de soufre de 0,2 à 4, de préférence 0,6 à 1,8 parties.

9. Mélanges vulcanisables, exempts de soufre ($S_8$) comprenant des charges et d'autres composants usuels, à base d'un ou plusieurs caoutchoucs naturels et/ou synthétiques, caractérisés en ce qu'ils comprennent de 0,3 à 10 parties de composé selon la revendication 1.

**Claims**

1. Bis-(2-ethylamino -4-diethylamino -s-triazin -6-yl)tetrasulfide.

2. A process for the production of the compound claimed in claim 1, characterized in that an alkaline aqueous solution of 2-ethylamino -4-diethylamino -6-mercaptotriazine is reacted in a 2-phase system with a solution of $S_2Cl_2$ in an inert organic solvent, in which the tetrasulfide formed is insoluble or only sparingly soluble, at temperatures of $< +10$ °C.

3. A process as claimed in claim 2, characterized in that alkali is used at least in the quantity required to form the alkali mercaptide or in a 5 to 10% larger quantity.

4. A process as claimed in claims 2 and 3, characterized in that a gasoline, petroleum ether or cyclohexane is used as solvent.

5. The use of the compound claimed in claim 1 as an accelerator in vulcanizable mixtures containing fillers, sulfur and other typical constituents and based on one or more natural and/or synthetic rubber(s) optionally containing a further accelerator and/or retarder and/or organosilane.

6. The use of 0,3 to 10 parts by weight of the compound claimed in claim 1 per 100 parts rubber as crosslinker in vulcanizable, sulfur ($S_8$)-free mixtures containing fillers and other typical constituents and based on one or more natural and/or synthetic rubber(s) optionally containing a further accelerator and/or retarder and/or organosilane.

7. Vulcanizable mixtures containing fillers, sulfur and other typical constituents and based on one or more natural and/or synthetic rubber(s), characterized in that they contain from 0,3 to 15 parts of the compound claimed in claim 1.

8. A vulcanizable mixture as claimed in claim 7, characterized in that it contains Vulkalent E[(N-trichloromethylthiophenesulfonyl)-benzene] and the compound claimed in claim 1 in a molar ratio of 1:1 for a sulfur dosage of 0,2 to 4 and preferably 0,6 to 1,8 parts.

9. Vulcanizable, sulfur ($S_8$)-free mixtures containing fillers and other typical constituents and based on one or more natural and/or synthetic rubbers, characterized in that they contain from 0,3 to 10 parts of the compound claimed in claim 1.